# EUROPEAN PATENT SPECIFICATION

(11) **EP 1 981 815 B1**
(45) Date of publication and mention of the grant of the patent: **14.10.2009**
(21) Application number: 07705077.1
(22) Date of filing: 30.01.2007
(51) Int. Cl.: C02F 1/26, C07C 227/22, C07C 227/42

(54) **TREATMENT OF AQUEOUS LIQUIDS AND THE PREPARATION OF ANTHRANILIC ACID**
BEHANDLUNG VON WÄSSRIGEN FLÜSSIGKEITEN UND HERSTELLUNG VON ANTHRANILSÄURE
TRAITEMENT DE LIQUIDES AQUEUX ET PREPARATION D'ACIDE ANTHRANILIQUE

(30) Priority: 03.02.2006 GB 0602227
(43) Date of publication of application: 22.10.2008
(73) Proprietor: Prom Limited, Surrey CR3 5UH (GB)
(72) Inventor: BERG, Carsten, DK-4791 Borre (DK)
(74) Representative: Westwood, Joanna
(86) International application number: PCT/GB2007/000313
(87) International publication number: WO 2007/088346

(56) References cited:
- DE-C- 55 988
- US-A- 2 653 971
- US-A- 4 276 433
- US-A- 4 925 565
- DATABASE WPI Week 199821 Derwent Publications Ltd., London, GB; AN 1998-234730 XP002439221 & JP 10 072417 A (NIPPON KAYAKU KK) 17 March 1998 (1998-03-17)

## Description

### Field of the invention

The present invention relates to an improved process for the formation of anthranilic acid.

### Background of the invention

Anthranilic acid is a raw material used in the preparation of the dye indigotin and, as its methyl ester, in the preparation of jasmine-like and orange blossom-like synthetic perfumes.

In United States patent US 4 276 433 (Kilpper et al.) a process is described for the continuous preparation of anthranilic acid by a two-stage reaction of an alkali metal phthalamate and/or an alkali metal phthalimidate with an alkali metal hypohalite, the first stage being carried out substantially adiabatically and both stages being carried out at high flow rates and different temperatures, wherein first the phthalimide and/or phthalamic acid is dissolved in a defined excess amount of alkali metal hydroxide solution and the solution is only then mixed, and reacted, with the hypohalite, without further addition of excess alkali. The products are starting materials for the preparation of dyes and scents.

DE 55 988 describes a process where after filtration copper acetate is added to the filtrate which is converted to the acid.

Generally, anthranilic acid is prepared by a continuous process in which a solution of an alkali metal salt of phthalamic acid is subjected to a Hofmann rearrangement, for example by treatment with sodium hypochlorite and sodium hydroxide. This leads to an anthranilic acid salt together with a carbonate salt as well as other inorganic salts, such as sodium chloride, in solution.

The final step is to add an acid such as sulphuric acid, whereby carbon dioxide is expelled leaving an aqueous slurry of anthranilic acid with the inorganic salts. While the slurry may be filtered to yield anthranilic acid, the filtrate still contains significant levels of anthranilic acid and inorganic salts.

As such, this filtrate is considered unsuitable for immediate discharge into waste water drainage systems, especially as the organic content thereof is so high, for example 10,000 mg/l, whereas regulations typically set a maximum organics content, expressed as the chemical oxygen demand (COD) of waste water at 500 mg/l.

Also the high salt level, which may for example be 12 weight percent, renders the filtrate unsuitable for bio-treatment. Also, loss of some of the anthranilic acid in the filtrate renders the yield of the process less than is desirable.

Other chemical processes also lead to waste water which contains high levels of organic material and inorganic salts and which, as a consequence is unsuitable for immediate discharge into waste water drainage systems.

There is therefore a need to provide a process for the treatment of such an aqueous liquid to render it more suitable for discharge into drainage systems, and in particular, in the case of the filtrate from an anthranilic acid process to remove and recover at least some of the anthranilic acid therefrom.

### SUMMARY OF THE INVENTION

We have discovered that these objectives can be achieved by a process which includes treating the aqueous liquid with a water-immiscible organic solvent to separate the organic material into an organic solvent-containing phase while the inorganic material remains in an aqueous phase.

We have surprisingly found that high salt levels in the aqueous phase speeds up the separation of the phases. Without wishing to be bound by theory we suspect that this may be due to the higher density of the aqueous phase and the reduced solubility of organics therein, when salt levels are higher.

Thus, according to a first aspect of the invention there is provided a process for forming an anthranilic acid which includes:
a) treating a mixture comprising a carbonate salt and an anthranilic acid salt of the formula (1) 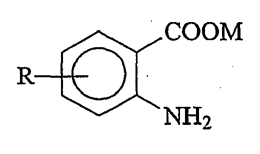 where R is selected from hydrogen and one or more substituted or unsubstituted hydrocarbon groups, and M is a univalent cation, with an inorganic acid in an aqueous medium to form an aqueous slurry comprising anthranilic acid and inorganic salts;
b) filtering the slurry to provide a residue comprising part of the anthranilic acid and a filtrate comprising the remainder of the anthranilic acid and the inorganic salts;
c) extracting further anthranilic acid from the filtrate with a water-immiscible organic solvent, whereby the further anthranilic acid is separated into an organic phase while the inorganic salts remain in an aqueous phase;
d) contacting the organic phase with an aqueous alkali to thereby transfer the further anthranilic acid from the organic phase to an aqueous phase as an anthranilic acid salt; and
e) adding the transferred anthranilic acid salt to the mixture of step a).

The mixture in step a) may additionally contain a chloride salt, especially if the mixture has been formed by a Hofmann rearrangement using a hypochlorite.

Usually, the inorganic salts comprise alkali metal salts, such as sodium salts, although the process is equally applicable where the salts are salts of other cations, especially salts of other alkali metals.

The inorganic acid preferably comprises sulphuric acid, although other inorganic acids such as hydrochloric acid may be used. Step a) is preferably carried out at a pH of less than 6.0, preferably.between 4.0 and 4.5.

The pH of the filtrate is ideally adjusted to between 4.0 and 4.5, preferably between 4.1 and 4.3 prior to extracting the further anthranilic acid, and is maintained at this pH during the extraction. This may be achieved by the addition of an alkali such as sodium hydroxide, or an acid such as sulphuric acid.

On the other hand, step d) is preferably carried out at a pH of above 6.6, preferably between 7.4 and 10.6. This can be achieved, for example, by contacting the organic phase, containing the further anthranilic acid, with aqueous alkali such as sodium hydroxide.

The aqueous phase from step d), containing the inorganic salts, may be further treated as follows. The pH is reduced to less than 2.85, preferably between 0.6 and 2.4, by the addition of an acid such as sulphuric acid and then contacting with an organic solvent, such as the same organic solvent used in step c), to extract any further organic material into the organic solvent. After separation, the aqueous phase can be subjected to treatment with Fenton's reagent (hydrogen peroxide / ferrous sulphate) to bring the chemical oxygen demand (COD) below 500 mg/l and then discharged to waste. The organic phase can be distilled to enable the organic solvent to be recycled. The residue may then be incinerated.

The invention is further illustrated in the following example and in the accompanying drawing, in which:

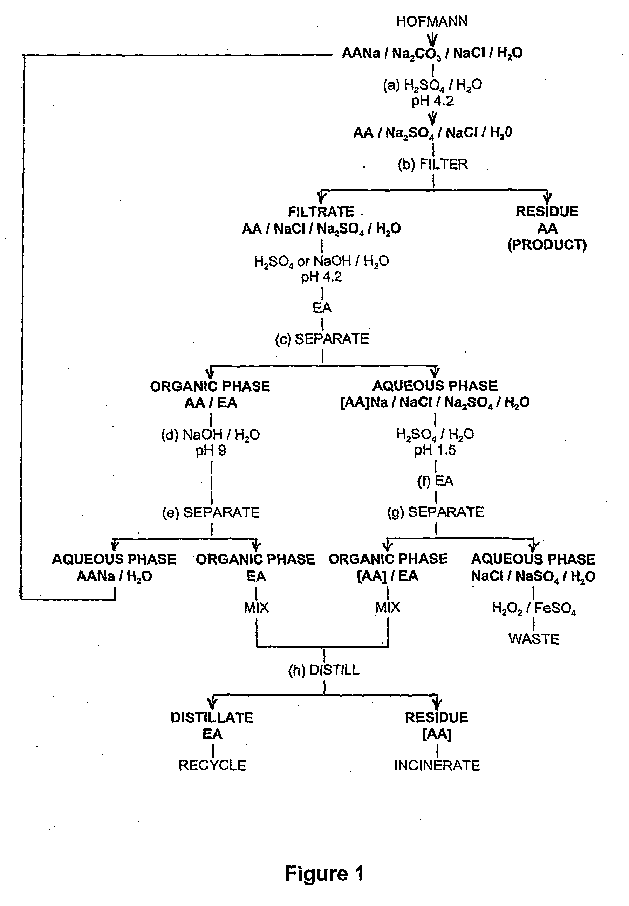

Figure 1 is a flow diagram illustrating an embodiment of the present invention.

### Detailed description of the drawing

An aqueous mixture comprising sodium carbonate, sodium chloride and the sodium salt of unsubstituted anthranilic acid (AANa in Figure 1) is formed from sodium phthalamidate by a Hofmann rearrangement using sodium hypochlorite in a manner known *per se.*

The mixture is treated at step (a) with sulphuric acid in an aqueous medium at a pH of about 4.2 to form an aqueous slurry comprising anthranilic acid (AA), sodium sulphate and sodium chloride.

The slurry is filtered at step (b) to provide a residue comprising part of the anthranilic acid as the product of the process. The aqueous filtrate comprises the remainder of the anthranilic acid and the sodium sulphate and chloride.

The pH of the filtrate is adjusted to about 4.2 by the addition of sodium hydroxide or sulphuric acid as appropriate. Further anthranilic acid is then extracted from the filtrate with ethyl acetate (EA), keeping the pH at 4.2 during extraction. The further anthranilic acid is separated at step (c) into an upper organic phase while the inorganic salts remain in a lower aqueous phase.

The upper organic phase is then contacted with aqueous sodium hydroxide at step (d) at a pH of about 9 to convert the further anthranilic acid (AA) in the upper organic phase to the sodium salt of anthranilic acid (AANa), which becomes transferred to the lower aqueous phase. The lower aqueous phase with the anthranilic acid salt is separated at step (e) and added to the Hofmann mixture of step (a), optionally after boiling to remove any carried over ethyl acetate. In this manner the overall yield of the anthranilic acid product is increased.

The lower aqueous phase from step (c), is waste water containing greater than 10 g/l of the inorganic salts and a small quantities of anthranilic acid and/or other organic materials ([AA]Na in Figure 1). As such it is unsuitable for discharge into a waste water drainage system. It is further treated as follows.

The pH is reduced to about 1.5 by the addition of aqueous sulphuric acid and it is then contacted with ethyl acetate (EA) in step (f) to extract any further organic material into the upper organic phase, while the inorganic salts remain in the lower aqueous phase. After separation at step (f), the lower aqueous phase is subjected to treatment with Fenton's reagent (hydrogen peroxide / ferrous sulphate) to oxidise any remaining organic material, more specifically to bring the chemical oxygen demand (COD) below 500 mg/l and then discharged to waste.

The upper organic phases separated from steps (e) and (f) are optionally now mixed and distilled to recover the ethyl acetate as the distillate. This recovered ethyl acetate is then reused. The residue ([AA]) from the distillation is then incinerated.

The invention will now be further illustrated in the following non-limiting example.

### Example

The details of this example are to be read in association with Figure 1.

The filtrate from the isolation (step (b) in Figure 1) of anthranilic acid through the continuous process where sodium phthalamidate was reacted with sodium hypochlorite and acidified (step (a)) with spent sulphuric acid to pH 4.2, contained 7.5% sodium sulphate and 4.1% sodium chloride. The major organic constituents were 4.4 g/kg anthranilic acid and 1.9 g/kg phthalic acid. The COD level was 18,200 mg/l.

300 g of the anthranilic acid filtrate was stirred with 300 g ethyl acetate for 30 min keeping the pH at 4.2, by adjusting with sulphuric acid. The organic phase was separated (step (c)) from the aqueous phase, which contained 0.18 g/kg anthranilic acid and 1.62 g/kg phthalic acid with a COD level of 3950 mg/l.

The organic phase from step (c) was stirred with water and 150 g sodium hydroxide for 15 min at pH 9 (step (d)). Anthranilic acid became transferred to the aqueous phase in an amount corresponding to 4.1 g/kg. The aqueous phase was boiled for a short time to expel any ethyl acetate, and was added to the final reaction mixture in the anthranilic acid preparation at the stage before isolation of the product.

The aqueous phase from step (c) was adjusted to pH 1.5 with sulphuric acid and was extracted with ethyl acetate for 30 min (step (f)). The organic phase was separated off (step (g)) and ethyl acetate was expelled from the aqueous phase which has a COD level of 1010 mg/kg.

50 g of the aqueous phase from step (g) was adjusted to pH 4.5 with sodium hydroxide and 0.53 g of a solution of ferrous sulphate, 5% as Fe(II), was added. With stirring 0.92 g of a 17% solution of hydrogen peroxide was slowly added and the mixture was stirred for 30 min. The pH was adjusted to 8.5 with sodium hydroxide and the separated iron hydroxide was filtered off.

The Fenton-treated aqueous phase had a COD level of 476 mg/l.

## Claims

1. A continuous process for forming an anthranilic acid which includes:
a) treating a mixture comprising a carbonate salt and an anthranilic acid salt of the formula (1) 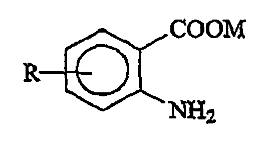 where R is selected from hydrogen and one or more substituted or unsubstituted hydrocarbon groups, and M is a univalent cation, with an inorganic acid in an aqueous medium to form an aqueous slurry comprising anthranilic acid and inorganic salts;
b) filtering said slurry to provide a residue comprising part of said anthranilic acid and a filtrate comprising the remainder of said anthranilic acid and said inorganic salts;
c) extracting further anthranilic acid from said filtrate with a water-immiscible organic solvent, whereby the further anthranilic acid is separated into an organic phase while the inorganic salts remain in an aqueous phase;
d) contacting said organic phase with an aqueous alkali to thereby transfer said further anthranilic acid from said organic phase to an aqueous phase as an anthranilic acid salt; and
e) adding the transferred anthranilic acid salt to the mixture of step a).

2. A process according to claim 1, wherein the mixture in step a) additionally contains a chloride salt.

3. A process according to claim 1 or 2, wherein the inorganic salts comprise alkali metal salts, such as sodium salts.

4. A process according to any preceding claim, wherein the inorganic acid comprises sulphuric acid.

5. A process according to any preceding claim, wherein step a) is carried out at a pH of less than 6.0, preferably between 4.0 and 4.5.

6. The process of any preceding claim, wherein the pH of said filtrate is adjusted to between 4.0 and 4.5, preferably between 4.1 and 4.3, prior to extracting said further anthranilic acid.

7. The process of any preceding claim, wherein step d) is carried out at a pH of above 6.6, preferably between 7.4 and 10.6.

8. The process of any preceding claim, wherein the organic solvent comprises an alkyl ester of a carboxylic acid, such as ethyl acetate or butyl acetate, a ketone such as methyl ethyl ketone, or an aromatic hydrocarbon such as toluene.

9. The process of any preceding claim, wherein the aqueous phase from step c), containing the inorganic salts, is further treated by adjusting the pH thereof to less than 2.85, preferably between 0.6 and 2.4, and then (f) contacting with an organic solvent to extract any further organic material into the organic solvent.

10. The process of claim 9, wherein after separation from the organic phase, the aqueous phase is treated with Fenton's reagent to bring the chemical oxygen demand (COD) thereof below 500 mg/l.

11. The process of claim 9 or 10, wherein the aqueous phase from step c) contains greater than 10 g/l inorganic salts, and the organic solvent of step f) comprises a volatile water-immiscible organic solvent, the process further comprising:
(g) separating an organic solvent-containing phase from a water-containing phase containing less than 0.5 g/l organic material;
(h) evaporating the organic solvent from the organic solvent-containing phase to leave a residue; and
(i) incinerating said residue.

12. The process of claim 11, wherein the evaporated organic solvent is recycled.

13. The process of any one of claims 11 or 12, wherein the organic solvent of step f) comprises an alkyl ester of a carboxylic acid, such as ethyl acetate or butyl acetate, a ketone such as methyl ethyl ketone, or an aromatic hydrocarbon such as toluene.

14. The process of any one of claims 11 to 13, wherein after separating from the organic solvent-containing phase the water-containing phase is discharged to waste.

## Patentansprüche

1. Kontinuierliches Verfahren zum Herstellen einer Anthranilsäure, indem man:
(a.) eine Mischung eines Carbonatsalzes und eines Salzes der Anthranilsäure gemäß der Formel (1) 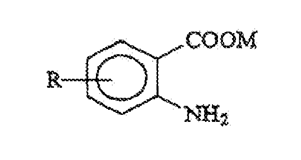 in der R aus Wasserstoff und einer oder mehr substituierten oder nicht substituierten Kohlenwasserstoffgruppen gewählt und M ein einwertiges Kation ist, mit einer anorganischen Säure in einem wässrigen Medium behandelt, um eine wässrige Aufschlämmung mit Anthranilsäure und anorganischen Salzen herzustellen;
(b.) die Aufschlämmung gefiltert wird, um einen Rückstand, der einen Teil der Anthranilsäure aufweist, und um ein Filtrat bereit zu stellen, welches den Säurerest der Anthranilsäure und die anorganischen Salze aufweist;
(c.) aus dem Filtrat mit einem mit Wasser nicht mischbaren organischen Lösungsmittel weitere Anthranilsäure extrahiert wird, wobei die weitere Anthranilsäure in einer organische Phase abgetrennt wird, die anorganischen Salze aber in einer wässrigen Phase verbleiben;
(d.) die organische Phase mit wässrigem Alkali kontaktiert wird, um so die weitere Anthranilsäure aus der organischen Phase als Salz der Anthranilsäure in eine wässrige Phase zu überführen; und
(e.) das überführte Salz der Anthranilsäure der Mischung des Schritts (a.) hinzugefügt wird.

2. Verfahren nach Anspruch 1, bei dem die Mischung im Schritt (a.) zusätzlich ein chloridsalz enthält.

3. Verfahren nach Anspruch 1 oder 2, bei dem die anorganischen Salze Alkalimetallsalze, wie bspw. Natriumsalze, umfassen.

4. Verfahren nach einem der vorgehenden Ansprüche, bei dem die anorganische Säure Schwefelsäure umfasst.

5. Verfahren nach einem der vorgehenden Ansprüche, bei dem der Schritt (a.) bei einem pH-Wert kleiner als 6,0, vorzugsweise zwischen 4,0 und 4,5 ausgeführt wird.

6. Verfahren nach einem der vorgehenden Ansprüche, bei dem vor dem Extrahieren der weiteren Anthranilsäure der pH-Wert des Filtrats auf einen Wert zwischen 4,0 und 4,6, vorzugsweise zwischen 4,1 und 4,3 eingestellt wird.

7. Verfahren nach einem der vorgehenden Ansprüche, bei dem der Schritt (d.) bei einem pH-Wert über 6,6, vorzugsweise zwischen 7,4 und 10,6 ausgeführt wird.

8. Verfahren nach einem der vorhergehenden Ansprüche, bei dem das organische Lösungsmittel einen Alkylester einer Carbonsäure, wie Ethyl- oder Butylacetat, ein Keton, wie Methylethylketon, oder einen aromatischen Kohlenwasserstoff, wie Toluol umfasst.

9. Verfahren nach einem der vorgehenden Ansprüche, bei dem die die anorganischen Salze enthaltende wässrige Phase aus dem Schritt (c.) weiter behandelt wird durch Einstellen ihres pH-Werts auf wenige als 2,85, vorzugsweise zwischen 0,6 und 2,4, und bei dem man dann
(f) mit einem organischen Lösungsmittel kontaktiert, um weitere organische Substanz in das organische Lösungsmittel zu extrahieren.

10. Verfahren nach Anspruch 9, bei dem nach dem Abtrennen der organischen Phase die wässrige Phase mit Fenton-Reagens behandelt, um deren chemischen Sauerstoffbedarf (COD) unter 500 mg/l zu bringen.

11. Verfahren nach Anspruch 9 oder 10, bei dem die wässrig Phase aus dem Schritt c.) mehr als 10 g/l anorganische Salze enthält und das organische Lösungsmittel des Schritts (f.) ein flüchtiges und mit Wasser nicht mischbares organisches Lösungsmittel umfasst, sowie weiterhin mit folgenden Schritten:
(g.) Trennen einer organisches Lösungsmittel von einer Wasser enthaltenden Phase, die wenige als 0,5 g/l organische Substanz enthält;
(h.) Verdampfen des organischen Lösungsmittels aus der das organische Lösungsmittel enthaltenden Phase, um einen Rückstand zu belassen; und
(i.) Verbrennen des Rückstands.

12. Verfahren nach Anspruch 12, bei dem das verdampfte organische Lösungsmittel recycelt wird.

13. Verfahren nach einem der Ansprüche 11 oder 12, bei dem das organische Lösungsmittel des Schritts (f.) einen Alkylester einer Carbonsäure wie Ethyl- oder Butylacetat, ein Keton wie Methylethylketon oder einen aromatischen Kohlenwasserstoff wie Toluol umfasst.

14. Verfahren nach einem der Ansprüche 11 bis 13, bei dem nach dem Abtrennen von der das organische Lösungsmittel enthaltenden Phase die Wasser enthaltende Phase entsorgt wird.

## Revendications

1. Procédé en continu pour former un acide anthranilique, qui comprend :
a) le traitement d'un mélange comprenant un sel carbonate et un sel d'acide anthranilique de formule (1) 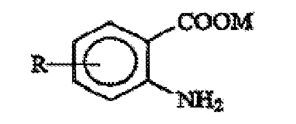 dans laquelle R est choisi entre hydrogène et un ou plusieurs groupes hydrocarbonés substitués ou non substitués, et M est un cation monovalent,
avec un acide inorganique dans un milieu aqueux pour former une suspension épaisse aqueuse comprenant de l'acide anthranilique et des sels inorganiques ;
b) la filtration de ladite suspension épaisse pour obtenir un résidu comprenant une partie dudit acide anthranilique et un filtrat comprenant le reste dudit acide anthranilique et lesdits sels inorganiques ;
c) l'extraction à nouveau de l'acide anthranilique à partir dudit filtrat avec un solvant organique non miscible à l'eau, ce par quoi l'acide anthranilique supplémentaire est séparé dans une phase organique tandis que les sels inorganiques restent dans une phase aqueuse ;
d) la mise en contact de ladite phase organique avec une solution d'un alcali aqueux de façon à transférer ainsi ledit acide anthranilique supplémentaire depuis ladite phase organique vers une phase aqueuse sous la forme d'un sel d'acide anthranilique ; et
e) l'ajout du sel d'acide anthranilique transféré au mélange de l'étape a.

2. Procédé selon la revendication 1, dans lequel le mélange dans l'étape a) contient de plus un sel chlorure.

3. Procédé selon la revendication 1 ou 2, dans lequel les sels inorganiques comprennent des sels de métal alcalin, tels que des sels de sodium.

4. Procédé selon l'une quelconque des revendications précédentes, dans lequel l'acide inorganique comprend de l'acide sulfurique.

5. Procédé selon l'une quelconque des revendications précédentes, dans lequel l'étape a) est mise en oeuvre à un pH inférieur à 6,0, de préférence compris entre 4,0 et 4,5.

6. Procédé selon l'une quelconque des revendications précédentes, dans lequel le pH dudit filtrat est ajusté entre 4,0 et 4,5, de préférence entre 4,1 et 4,3, avant l'extraction dudit acide anthranilique supplémentaire.

7. Procédé selon l'une quelconque des revendications précédentes, dans lequel l'étape d) est mise en oeuvre à un pH supérieur à 6,6, de préférence compris entre 7,4 et 10,6.

8. Procédé selon l'une quelconque des revendications précédentes, dans lequel le solvant organique comprend un ester alkylique d'un acide carboxylique, tel que l'acétate d'éthyle ou l'acétate de butyle, une cétone telle que la méthyléthylcétone, ou un hydrocarbure aromatique tel que le toluène.

9. Procédé selon l'une quelconque des revendications précédentes, dans lequel la phase aqueuse obtenue dans l'étape c), contenant les sels inorganiques, est encore traitée par ajustement du pH de celle-ci à une valeur inférieure à 2,85, de préférence comprise entre 0,6 et 2,4, et est ensuite (f) mise en contact avec un solvant organique pour extraire toute matière organique supplémentaire dans le solvant organique.

10. Procédé selon la revendication 9, dans lequel, après séparation d'avec la phase organique, la phase aqueuse est traitée avec du réactif de Fenton pour porter la demande en oxygène chimique (COD) de celle-ci en-deçà de 500 mg/l.

11. Procédé selon la revendication 9 ou 10, dans lequel la phase aqueuse obtenue dans l'étape c) contient plus de 10 g/l de sels inorganiques, et le solvant organique de l'étape f) comprend un solvant organique volatil non miscible à l'eau, le procédé comprenant en outre :
(g) la séparation d'une phase contenant le solvant organique d'avec une phase aqueuse contenant moins de 0,5 g/l de matière organique ;
(h) l'évaporation du solvant organique de la phase contenant le solvant organique pour laisser un résidu ; et
(i) l'incinération dudit résidu.

12. Procédé selon la revendication 11, dans lequel le solvant organique évaporé est recyclé.

13. Procédé selon l'une quelconque des revendications 11 et 12, dans lequel le solvant organique de l'étape f) comprend un ester alkylique d'un acide carboxylique, tel que l'acétate d'éthyle ou l'acétate de butyle, une cétone telle que la méthyléthylcétone, ou un hydrocarbure aromatique tel que le toluène.

14. Procédé selon l'une quelconque des revendications 11 à 13, dans lequel, après séparation d'avec la phase contenant le solvant organique, la phase aqueuse est mise au rebut.
